# EUROPEAN PATENT SPECIFICATION

(11) **EP 4 302 084 B1**
(45) Date of publication and mention of the grant of the patent: **17.12.2025**
(21) Application number: 22717425.7
(22) Date of filing: 28.02.2022
(51) Int. Cl.: G01N 27/447, G01N 33/68, C07K 14/005

(54) **CAPILLARY ELECTROPHORESIS METHODS FOR QUANTIFYING VIRAL SPECIES**
KAPILLARELEKTROPHORESEVERFAHREN ZUR QUANTIFIZIERUNG VON VIRUSSPEZIES
PROCÉDÉS D'ÉLECTROPHORÈSE CAPILLAIRE POUR LA QUANTIFICATION D'ESPÈCES VIRALES

(30) Priority: 03.03.2021 US 202163155891 P
(43) Date of publication of application: 10.01.2024
(73) Proprietor: DH Technologies Development Pte. Ltd., Singapore 739256 (SG)
(72) Inventor: LI, Tingting, Concord, Ontario L4K 4V8 (CA); YOWANTO, Handy, Concord, Ontario L4K 4V8 (CA); GUTTMAN, Andras, Concord, Ontario L4K 4V8 (CA)
(74) Representative: Maiwald GmbH
(86) International application number: PCT/IB2022/051754
(87) International publication number: WO 2022/185185

(56) References cited:
- US-B2- 7 993 647
- LI TINGTING ET AL: "Ultrahigh Sensitivity Analysis of Adeno-associated Virus (AAV) Capsid Proteins by Sodium Dodecyl Sulphate Capillary Gel Electrophoresis", 23 November 2020 (2020-11-23), XP055859350, Retrieved from the Internet <URL:https://www.labmate-online.com/article/bioanalytical/40/sciex/ultrahigh-sensitivity-analysis-of-adeno-associated-virus-aavbr-capsid-proteins-by-sodium-dodecyl-sulphate-capillary-gel-electrophoresis/2852> [retrieved on 20211109]
- LI TINGTING ET AL: "Characterization of Adeno-Associated Virus (AAV) using capillary electrophoresis", PEGS EUROPE 2019, 18 November 2019 (2019-11-18), pages 1 - 1, XP055828863, ISBN: 978-3-527-33374-5, Retrieved from the Internet <URL:https://www.researchgate.net/publication/342978276_Characterization_of_Adeno-Associated_Virus_AAV_Using_Capillary_Electrophoresis> DOI: 10.1002/9783527678129.assep035
- DOUGLAS B CRAIG ET AL: "Determination of picomolar concentrations of proteins using novel amino reactive chameleon labels and capillary electrophoresis laser-induced fluorescence detection", ELECTROPHORESIS, VERLAG CHEMIE, HOBOKEN, USA, vol. 26, no. 11, 9 May 2005 (2005-05-09), pages 2208 - 2213, XP071497712, ISSN: 0173-0835, DOI: 10.1002/ELPS.200410332
- ANONYMOUS: "Capillary electrophoresis kits", 1 January 2020 (2020-01-01), XP055929118, Retrieved from the Internet <URL:https://lcms.cz/labrulez-bucket-strapi-h3hsga3/CE_Kit_de23cc824b/CE-Kit.pdf> [retrieved on 20220608]

## Description

### RELATED APPLICATIONS

The present patent application claims the priority benefit of U.S. Provisional Patent Application Ser. No. 63/155,891, filed March 3, 2021.

### BACKGROUND

The various challenges during viral species production include virus stability, the virus' toxicity to the host cell lines, and limited ability to properly characterize the active virus product. As a result, monitoring product quality is critical. Titer is one of the critical quality attributes (CQA) for viral species production from lab scale to GMP manufacturing. Quantitation (titer determination) of a viral species to ensure efficient expression is usually based on the quantification of a protein of interest. An enzyme-linked immunosorbent assay (ELISA) is the traditional method for viral species titer determination. However, this method measures both virus-associated protein(s) of interest and possible false positives with similar epitopes, resulting in an overestimation of the titer. This calls for an orthogonal method to be used in combination with the regularly used screening ELISA test, such as a separation-based technique. The latter can be used to recognize false positives and provide more accurate titer and concomitantly utilized for profile-based purity assessment. Guzman, N.A. and D.E. Guzman, A Two-Dimensional Affinity Capture and Separation Mini-Platform for the Isolation, Enrichment, and Quantification of Biomarkers and Its Potential Use for Liquid Biopsy. Biomedicines, 2020. 8(8). Application of electrophoresis for the detection of Adeno-associated Virus (AVV) has been described by Tingting Li et al. , 2020-11-23, at LABMATE Online: "Ultrahigh Sensitivity Analysis of Adeno-associated Virus (AAV) Capsid Proteins by Sodium Dodecyl Sulphate Capillary Gel Electrophoresis"; https:// www.labmate-online.com/article/bioanalytical/40/sciex/ultrahigh-sensitivity-analysis-of-adeno-associated-virus-aavbr-capsid-proteins-by-sodium-dodecyl-sulphate-capillary-gel-electrophoresis/2852.

### SUMMARY

The inventors have recognized the need for high precision viral species titer determination and its protein profiling based purity assessment. The claimed and described approach offers an automated analysis of the viral species proteome with high resolution, excellent quantitation capability, and great reproducibility. An easily applicable two-step sample preparation and fluorescent labeling protocol is also described.

### BRIEF DESCRIPTION OF THE FIGURES

FIG. 1 illustrates a lentivirus structure. Titer determination is accomplished by quantifying the p24 capsid protein.
FIG. 2 depicts a sodium dodecyl sulfate capillary gel electrophoresis of pyrylium dye labeled lentivirus proteome. Conditions: EZ-CE cartridge with 30 cm total length (20 cm to the detection point, 50 µm ID) fused silica capillary filled with a buffer comprising a polymer matrix, E=500V/cm, temperature: 25°C, Injection parameters: water pre-injection for 0.4 min at 20 psi followed by sample injection for 1 min at 5 kV.
FIGs. 3A - 3D depict the p24 determination of sodium dodecyl sulfate-capillary gel electrophoresis (SDS-CGE) analysis of the pyrylium dye labeled lentivirus proteome. The circled peak is the p24 protein for titer determination. The increasing amounts of spiked p24 are shown at the corresponding traces. The lentivirus concentration of FIGs. 3A and 3B is 1.0X10⁹ TU/mL and the lentivirus concentration of FIGs. 3C and 3D is 1.5X10⁸ TU/mL. Separation conditions were the same as in FIG. 2.
FIGs. 4A and 4B depict the linearity and limit of quantification based on the SDS-CGE analysis of the p24 protein.
FIGs. 5A and 5B depict the repeatability and reproducibility of the lentivirus titer determination detection based on the SDS-CGE analysis of the p24 protein.
FIGs. 6A and 6B depict repeatability and reproducibility the lentivirus titer determination detection of six different sample preparation based on the SDS-CGE analysis of the p24 protein.
FIG. 7A depicts the standard curve of a labeled viral protein component quantified on three separate days. FIG. 7B depicts a superimposed p24 standard at different concentrations. FIG. 7C depicts the average of the standard curves prepared and analyzed across three different days with the corresponding error bars. Separation conditions were the same as in FIG. 2.
FIG. 8 shows initial conditions in accordance with one aspect of the disclosure.
FIG. 9 shows LIF detector initial conditions in accordance with one aspect of the disclosure.
FIG. 10 shows a time program for conditioning in accordance with one aspect of the disclosure.
FIG. 11 shows a time program for separation in accordance with one aspect of the disclosure.

### DETAILED DESCRIPTION

It is to be understood that this disclosure is not limited to the particular methodology, protocols, and reagents described herein and as such may vary.

As used herein and in the appended claims, the singular forms "a," "an," and "the" include plural reference unless the context clearly indicates otherwise. The singular form "a", "an" and "the" include plural referents unless the context clearly dictates otherwise. These articles refer to one or to more than one (i.e., to at least one). The term "and/or" means any one or more of the items in the list joined by "and/or". As an example, "x and/or y" means any element of the three-element set {(x), (y), (x, y)}. In other words, "x and/or y" means "one or both of x and y". As another example, "x, y, and/or z" means any element of the seven-element set {(x), (y), (z), (x, y), (x, z), (y, z), (x, y, z)}. In other words, "x, y and/or z" means "one or more of x, y and z".

The term "about" is used in connection with a numerical value throughout the specification and the claims denote an interval of accuracy, familiar and acceptable to a person skilled in the art. In general, such an interval of accuracy is +/-10%.

The term "exemplary" means serving as a non-limiting example, instance, or illustration. As utilized herein, the terms "e.g.," and "for example" set off lists of one or more non-limiting aspects, examples, instances, or illustrations.

The term "low ng/mL range" includes amounts from about 50 ng/mL -about 5000 ng/mL. The term "sub ng/mL range" includes amounts from about 0.01 ng/mL - about 49.99 ng/mL.

Where ranges are given, endpoints are included. Furthermore, unless otherwise indicated or otherwise evident from the context and understanding of one of ordinary skill in the art, values that are expressed as ranges can assume any specific value or subrange within the stated ranges in different embodiments of the disclosure, to the tenth of the unit of the lower limit of the range, unless the context clearly dictates otherwise.

Unless defined otherwise, all technical and scientific terms used herein have the same meaning as commonly understood to one of ordinary skill in the art to which this disclosure belongs.

Disclosed are methods for a high-performance sodium dodecyl sulfate capillary gel electrophoresis based assay, using a capillary electrophoresis platform in conjunction with sodium dodecyl sulfate-molecular weight protein analysis. Embodiments of this disclosure can be used for highly precise viral titer determination and profiling. The disclosed methods allow for the separation of proteins in the size range of about 10 kDa to about 225 kDa with high resolution, excellent quantitation capability, and great reproducibility. In addition to the separation methods, the method further comprises steps for denaturing and labeling a viral species for quantitative determination of a protein of interest using a detectable dye.

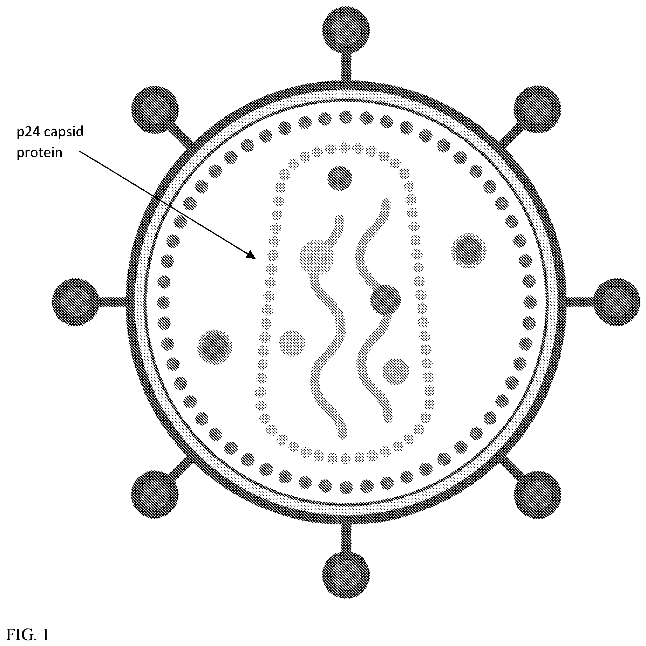

Lentivirus (depicted in FIG. 1) can integrate large DNA molecules into host cells, thus representing one of the most efficient gene delivery vehicles for transduction. Cockrell, A.S. and T. Kafri, Gene delivery by lentivirus vectors. Mol Biotechnol, 2007. 36(3): p. 184-204. For this particular application, lentivirus has several advantages, including its ability to infect non-dividing and dividing cells. For clinical therapeutic applications, the stable and long term transgene expression of these retroviruses represent a great advantage. Anguela, X.M. and K.A. High, Entering the Modern Era of Gene Therapy. Annu Rev Med, 2019. 70: p. 273-288. The proteome of the lentivirus comprises five important structural and several non-structural proteins. The vital structural proteins are gp120 surface envelope protein (120 kDa), gp41 transmembrane envelope protein (41 kDa), p24 capsid protein (24 kDa), p17 matrix protein (17 kDa), and the p7/P9 nucleocapsid protein (7-11 kDa). The two envelope proteins of gp120 and gp41 are highly glycosylated. This post-translational modification (PTM) apparently conceals important antigenic sites, thus helps the virus to avoid recognition by the immune system of the host. The p24 protein is the building block of the lentivirus capsid, and approximately 2000 of them assemble the full capsid.

Disclosed is a method for quantifying a viral species, the method comprising the steps of preparing a labeled viral protein component by incubating a detectable dye with a virial species in the presence of sodium dodecyl sulfate (SDS); loading the labeled viral protein component onto a capillary electrophoresis (CE) capillary, wherein the CE capillary is filled with a buffer comprising a polymer matrix; applying a separation voltage to the CE capillary; detecting at least one labeled viral protein component with a detector, thereby producing a corresponding set of values; and quantifying a protein of interest in the viral protein component using the corresponding set of values.

In an embodiment where the viral species is a lentivirus, the protein component detected is a structural protein, such as a nucleocapsid protein, capsid protein, matrix protein, or envelope protein. In an embodiment, the capsid protein is a p24 capsid protein. In some embodiments, the protein component detected is a non-structural protein or a residual protein.

In an embodiment, the method comprises an efficient two-step sample preparation protocol, including the denaturation and covalent labeling of the protein of interest with a detectable dye. The detectable dye may be a fluorescent dye, and includes dyes that independently have an absorption wavelength and emission wavelength of between about 480 nm and about 740 nm, alternatively about 490 nm, alternatively about 500 nm, alternatively about 510 nm, alternatively about 520 nm, alternatively about 530 nm, alternatively about 540 nm, alternatively about 550 nm, alternatively about 560 nm, alternatively about 570 nm, alternatively about 580 nm, alternatively about 590 nm, alternatively about 600 nm, alternatively about 610 nm, alternatively about 620 nm, alternatively about 630 nm, alternatively about 640 nm, alternatively about 650 nm, alternatively about 660 nm, alternatively about 670 nm, alternatively about 680 nm, alternatively about 690 nm, alternatively about 700 nm, alternatively about 710 nm, alternatively about 720 nm, alternatively about 730 nm. Non-limiting examples of the detectable dye include a pyrylium-based dye and an amine-reactive pyrylium dye. Pyrylium dyes fluoresce when the dye reacts with proteins. Amine-reactive pyrylium dye fluoresce when the dye readily reacts with the primary amines of the polypeptide backbone of proteins and features very low (<1%) fluorescence in its unconjugated format, thus, decreasing baseline noise and increasing detection limit. The use of pyrylium-based dyes also retains a protein's natural charge state.

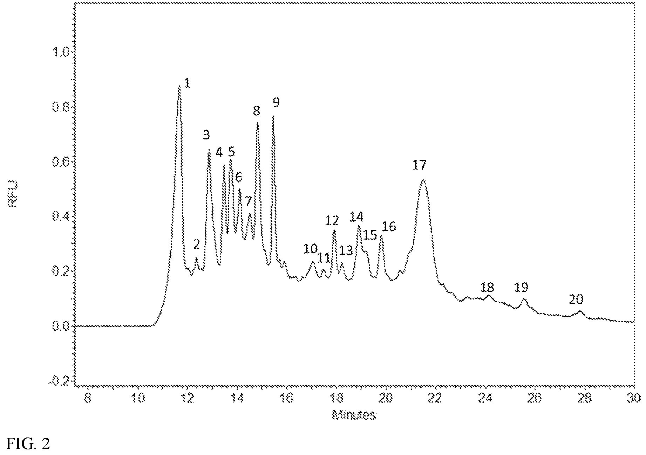

In an exemplary embodiment, a lentivirus proteome is labeled with a pyrylium dye and was then subject to quantitative sodium dodecyl sulfate capillary gel electrophoresis analysis. The resulting electropherogram is shown in FIG. 2 and features over 20 peaks. Since most of the lentivirus proteins possess various post-translational modifications (PTMs), such as glycosylation, standard curve based molecular mass assessment using the sizing ladder is not considered adequate.

The detectable dye is incubated with the viral species for a time sufficient to allow for the formation of a labeled viral protein component. In various embodiments, the viral species is incubated for at least 2 minutes, alternatively at least 5 minutes, alternatively at least 10 minutes, alternatively at least 15 minutes, alternatively at least 20 minutes, alternatively at least 25 minutes, alternatively at least 30 minutes, alternatively at least 35 minutes, alternatively at least 40 minutes, alternatively at least 45 minutes, alternatively at least 50 minutes, alternatively at least 55 minutes, alternatively at least 60 minutes.

The detectable dye is also incubated with the viral species at a temperature sufficient to allow for the formation of a labeled viral protein component. In various embodiments, the viral species is incubated at a temperature between about 40°C to about 90°C, alternatively at a temperature between about 45°C to about 85°C, alternatively at a temperature between about 50°C to about 80°C, alternatively at a temperature between about 55°C to about 78°C, alternatively at a temperature between about 60°C to about 77°C, alternatively at a temperature between about 65°C to about 75°C, alternatively at a temperature between about 68°C to about 74°C, alternatively at a temperature between about 69°C to about 73°C, alternatively at a temperature of about 70°C.

The labeled viral protein component is loaded onto a capillary electrophoresis (CE) capillary. The CE capillary may be filled with a buffer comprising a polymer matrix or gel buffer prior to applying a separation voltage and/or loading the labeled viral protein component. In some embodiments, the buffer comprising a polymer matrix or gel buffer is placed into a buffer vial. These buffer vials may be placed into buffer trays. In some embodiments, the buffer comprising a polymer matrix or gel buffer may comprise additional components to facilitate the separation of the protein and/or protein components of interest. Non-limiting examples of a suitable polymer matrix include crosslinked polymer, linear polymers, slightly branched polymers, linear polyacrylamide, polyethylene oxide, polyethylene glycol, dextran, and pullulan.

A separation voltage is applied to the CE capillary, and the labeled viral protein components are moved towards a detector. The detector can be a UV detector or a fluorescence detector, such as a laser induced fluorescence (LIF) detector, a lamp-based fluorescence detector, or a native fluorescence detector. The desired quantitation sensitivity will determine the type of detector used. LIF detection offers the benefit of about a 100-fold increase in sensitivity, yet it also requires additional sample manipulation.

Once the labeled viral protein components are detected, a corresponding set of values is generated. In an embodiment, these values may be plotted on an electropherogram. Once plotted on an electropherogram, at least one viral protein component from the labeled protein sample may be identified (e.g., qualitative assay). Qualitative analysis may also be used to estimate the molecular weights of the protein components. In various embodiments, the limits of detection are in the low ng/mL range, alternatively in the sub ng/mL range.

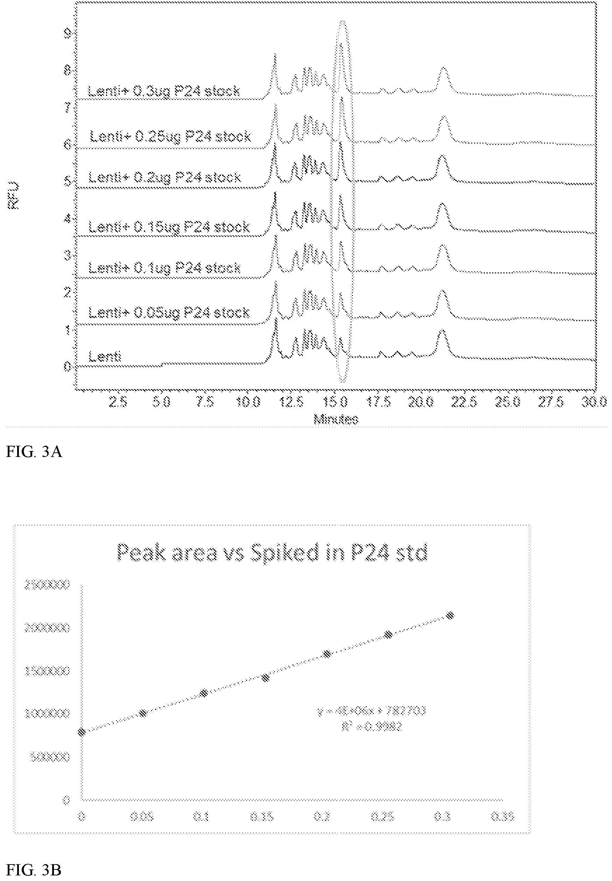

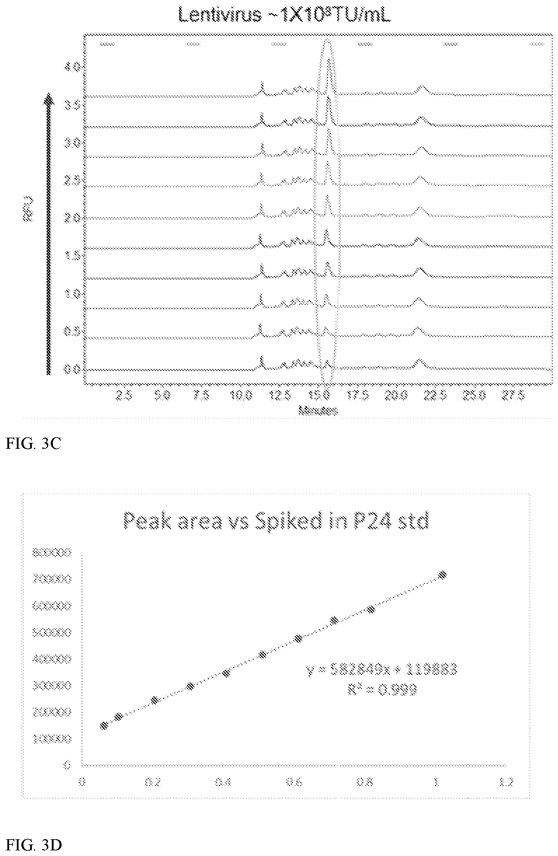

In an embodiment, the corresponding set of values is used to quantitate the viral protein components and/or used for viral titer determination. In an exemplary embodiment, the peak corresponding to the p24 protein for titer determination was determined by spiking the sample with a commercially available standard. FIGs. 3A and 3C compare the original separation trace with six spiked SDS-CGE electropherograms reflecting the effect of the increasing amount of the p24 protein from 0.05 µg to 0.3 µg (FIG. 3A) or from 0.003 µg to 0.051 µg (FIG. 3C) . In this embodiment peak 9 was identified as the p24 protein, later used for titer determination, and the resulting profile can be readily used for batch to batch reproducibility and purity assessment. This embodiment also illustrates the excellent reproducibility of the CGE-SDS method, even with various amounts of p24 in the samples proving that the quantitative variation of the p24 protein content did not affect profiling repeatability. In FIG. 3A differing amounts of a p24 standard were spiked into a lentivirus sample with concentration at 1.0X10⁹ TU/mL. FIG. 3B shows excellent linearity of the peak area of the identified p24 peak versus the amount of the p24 standard spiked in. In another aspect, the p24 protein standard was spiked into lentivirus sample at a lower concentration (1X10⁸ TU/mL) (FIG. 3C). The linearity of the peak area vs the spiked in amount of p24 is show in FIG. 3D. With the identification of the p24 peak and its good linearity of the standard addition studies, the p24 is then used for lentivirus titer determination.

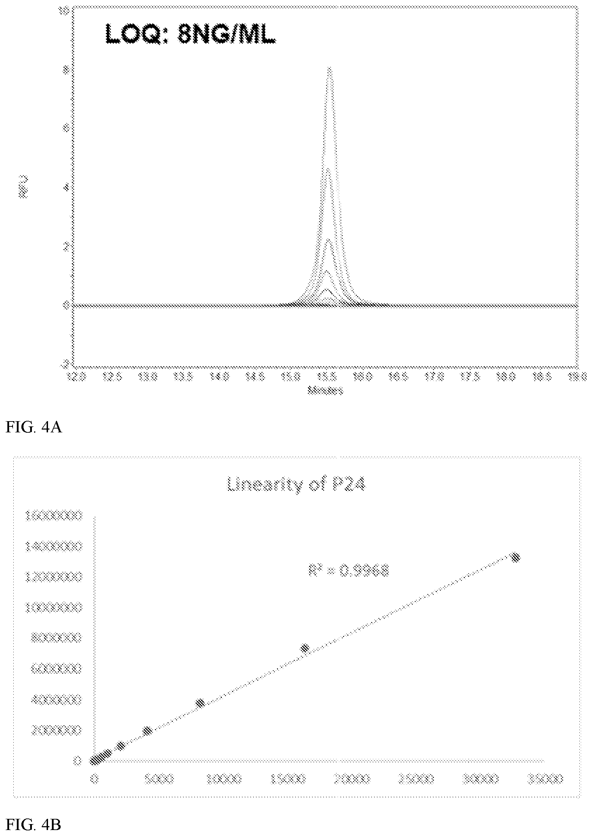

The limit of quantitation (LOQ) values were determined by injecting an increasing amount of standalone p24 standard into the SDS-MW gel-filled capillary column. In various embodiments, the limit of quantification is in a low ng/mL range or is in a sub ng/mL range. As shown in FIGs. 4A and 4B, the limit of quantification is 8 ng/mL.

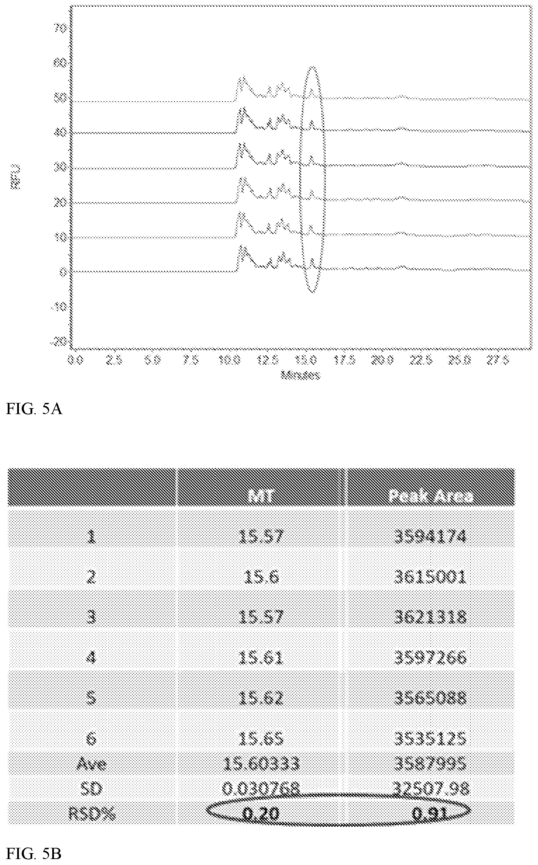

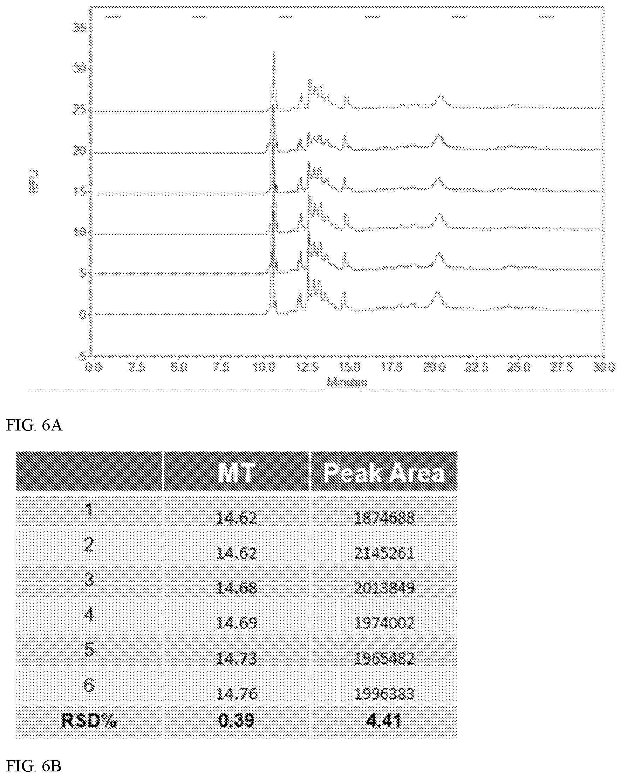

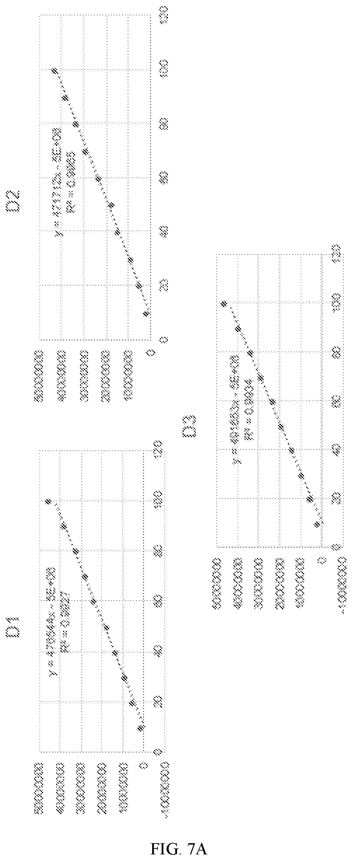

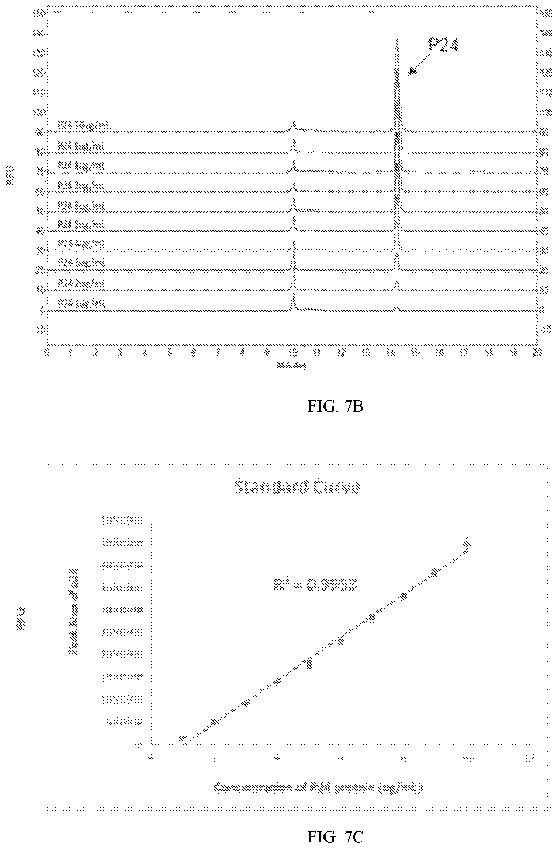

The disclosed methods have several advantages over conventional titer methods including, sensitive titer determination based on precise quantification of the separated protein peak, easy batch to batch reproducibility and purity assessment based on the resulting proteome profile, efficient two-step sample preparation protocol including denaturation and covalent detectable dye labeling of the viral species proteome with no sample cleanup requirement, excellent repeatability with 0.20 % RSD on migration time and less than 0.91 % on Corrected Peak Area% for six consecutive injections (FIGs. 5A and 5B), excellent repeatability with no more than 1 % RSD on migration time and less than 5.00 % on Corrected Peak Area% for six sample preparations (FIGs. 6A and 6B), good titer determination detection linearity (r² = 0.9953) in the concentration range of 1 µg/mL - 10 µg/mL (FIGs. 7A, 7B and 7C). These advantages are present even when multiple different labeled viral protein components are quantified or are quantified on separate days. FIGs. 7A and 7B show the standard curve of a labeled viral protein component quantified on three separate days (FIG. 7A) and the average of these standard curves (FIG. 7C). As illustrated this method still provided for good titer determination detection linearity (r² = 0.9953).

Table 1 shows the titer determination of a lentiviral vector sample across 3 days using the average standard curve as shown in FIG. 7C and compares to the titer determined by the ELISA P24 kit.

**Table 1**

| | **Titer (TU/mL)** |
|---|---|
| Titer calculated_D1 | 1.45 X10⁹ |
| Titer calculated_D2 | 1.45X10⁹ |
| Titer calculated_D3 | 1.46X10⁹ |
| Titer from vendor (ELISA P24 kit) | 1.5X10⁹ |

### EXAMPLES

### Example 1: Exemplary sample preparation and analysis method

### Materials and Methods

Materials: Pre-made lentivirus (LV-CAG-GFP, PN SL100270) was from SignaGen Laboratories (Rockville, MD), and the HIV1 p24 protein (PN ab127888) was purchased from Abcam (Cambridge, UK). The Chromeo P503 dye (PN 15106) was from Active Motif (Carlsbad, CA). Phosphate Buffered Saline (PBS) 10x bioreagent suitable for cell culture (PN P5493-1L) was from Sigma-Aldrich (St Louis, MO). The SDS-MW Analysis Kit (PN 390953) was from SCIEX (Framingham, MA), including the SDS-MW gel buffer, the acidic and basic wash solutions (0.1 N HCl and 0.1 N NaOH) as well as the SDS-MW sample buffer of 100 mM Tris-HCl (pH 9.0) with 1%SDS.

### Sample Preparation

Preparation of the Chromeo P503 working solution: 1 mg of Chromeo P503 dye (lyophilized powder) was reconstituted in 1 mL of methanol. After reconstitution, the dye label can be stored at 2-8°C for six months according to the manufacturer's instructions. Sample Preparation: 5 µL of lentivirus sample solution (1.5X10⁹ TU/mL) was mixed with 5 µL of sample preparation solution provided in the SDS-MW Analysis Kit and incubated at 70 °C for 10 minutes followed by mixing with 0.5 µL of Chromeo P503 dye working solution and incubated again for another 10 minutes at 70 °C. After cooling down to room temperature, 39.5µL of DI water was added to the reaction mixture, and the diluted sample was transferred to the sample vial for SDS-CGE-LIF analysis.

### Capillary Electrophoresis

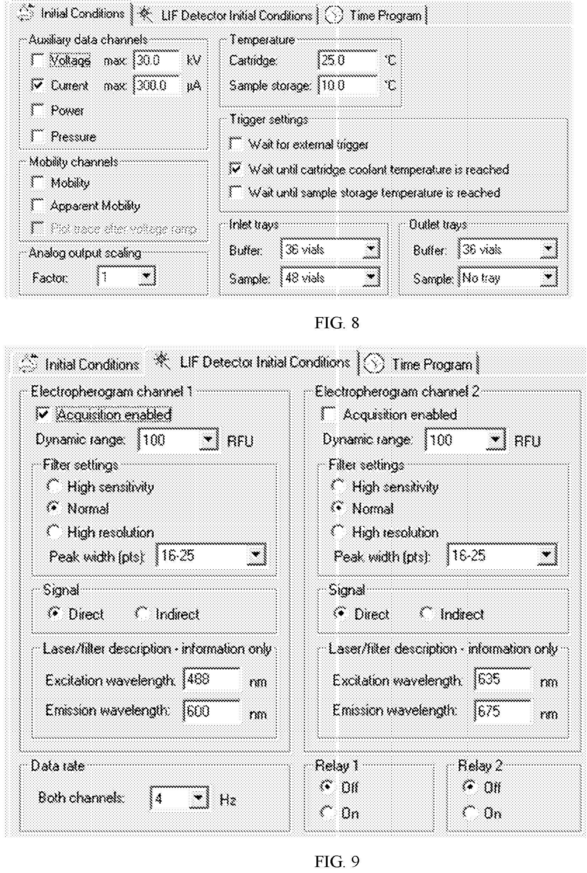

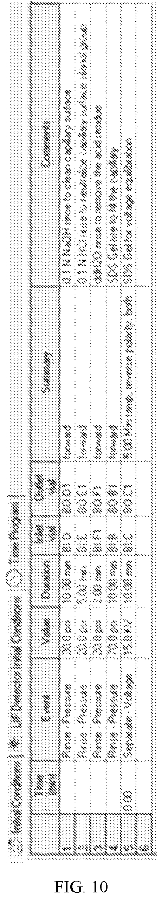

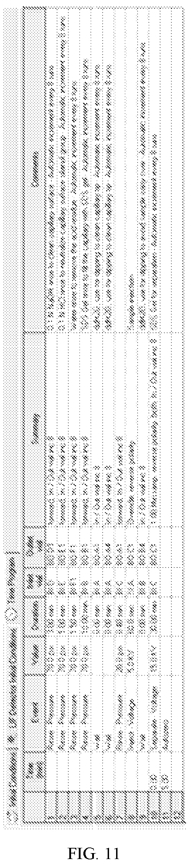

A PA800 Plus Pharmaceutical Analysis CE system (SCIEX) equipped with a laser-induced fluorescence (LIF) detector with a 488 nm solid-state laser and a 600 nm emission filter was used for all separations. The pre-assembled EZ-CE capillary cartridge (SCIEX, PN A55625) had a bare fused-silica capillary with 50 µm I.D. and 30 cm total length (20 cm effective length). The SCIEX universal vials (PN A62251), universal vial caps (PN A62250), and PCR vials (PN 144709) were used for sample loading with the following injection parameters: water stacking at 20.0psi for 0.40min and injection at 5kV for 60 seconds. Data acquisition and analysis were performed using 32 Karat^{™} software 10. The detailed information of condition, separation, and shutdown methods are shown in FIGs. 9-11.

### Example 2: Titer of a Lentivirus sample.

The titer of the lentivirus sample was determined by using the detection linearity plot in FIG. 7B and found to be 1.45 x 10⁹ TU/mL, 1.45 x 10⁹ TU/mL, and 1.46 x 10⁹ TU/mL as depicted in Table 2 during three consecutive days of analysis.

**Table 2. Lentivirus titer determined by SDS-CGE and ELISA.**

| | **Titer (TU/mL)** |
|---|---|
| Titer measured by SDS-CGE_Day1 | 1.45 X10⁹ |
| Titer measured by SDS-CGE_Day2 | 1.45x 10⁹ |
| Titer measured by SDS-CGE_Day3 | 1.46 x 10⁹ |
| Titer from vendor (ELISA P24 kit) | 1.5X10⁹ |

The titer value measured by ELISA assay was 1.5 x 10⁹ TU/mL (also shown in Table 1). As a first approximation, we consider this apparent difference in the titer values between the SDS-CGE and ELISA assays of an overestimation of the latter as the ELISA is prone to detect similar epitope proteins in addition to the target protein. This assumption was supported by the complicated profile of the lentivirus proteome (FIG. 2), i.e., the presence of all other proteins in the mixture possibly interfering with the ELISA. The SDS-CGE assay, on the other hand, provided the actual titer value of the sample solely based on quantitating the peak corresponding to the p24 protein (circled peak in FIG. 2), i.e., without any interference of other proteins in the sample. In addition, the resulting profile can be readily used for batch to batch purity assessment.

## Claims

1. A method for quantifying a viral species, the method comprising the steps of:
preparing at least one labeled viral protein component by incubating a detectable dye with a virial species in the presence of sodium dodecyl sulfate (SDS);
loading the labeled viral protein component onto a capillary electrophoresis (CE) capillary, wherein the CE capillary is filled with a buffer comprising a polymer matrix;
applying a separation voltage to the CE capillary;
detecting at least one labeled viral protein component with a detector, thereby producing a corresponding set of values; and
quantifying a protein of interest in the viral protein component using the corresponding set of values,
**characterized in that** the viral species is a retrovirus.

2. The method of claim 1, wherein the viral species is a lentivirus.

3. The method of claim 2, wherein the viral protein component is a structural protein, non-structural protein, or a residual protein.

4. The method of claim 3, wherein the viral protein component is a structural protein selected from the group consisting of nucleocapsid protein, capsid protein, matrix protein, and envelope protein.

5. The method of claim 3 or claim 4, wherein the structural protein is a p24 capsid protein.

6. The method of any one of the preceding claims, wherein the viral species is incubated at a temperature between about 40°C to about 90°C, alternatively at a temperature between about 45°C to about 85°C, alternatively at a temperature between about 50°C to about 80°C, alternatively at a temperature between about 55°C to about 78°C, alternatively at a temperature between about 60°C to about 77°C, alternatively at a temperature between about 65°C to about 75°C, alternatively at a temperature between about 68°C to about 74°C, alternatively at a temperature between about 69°C to about 73°C, alternatively at a temperature of about 70°C.

7. The method of any one of the preceding claims, wherein the viral species is incubated for at least 2 minutes, alternatively at least 5 minutes, alternatively at least 10 minutes, alternatively at least 15 minutes, alternatively at least 20 minutes, alternatively at least 25 minutes, alternatively at least 30 minutes, alternatively at least 35 minutes, alternatively at least 40 minutes, alternatively at least 45 minutes, alternatively at least 50 minutes, alternatively at least 55 minutes, alternatively at least 60 minutes.

8. The method of any of the preceding claims, wherein the detectable dye is a fluorescent dye.

9. The method of claim 8, wherein the detectable dye has an absorption wavelength and an emission wavelength of between about 480 nm and about 750 nm.

10. The method of claim 8 or claim 9, wherein the detectable dye is a pyrylium-based dye.

11. The method of claim 10, wherein the detectable dye is an amine-reactive pyrylium dye.

12. The method of any of the preceding claims, wherein the polymer matrix is selected from the group consisting of crosslinked polymer, linear polymers, slightly branched polymers, linear polyacrylamide, polyethylene oxide, polyethylene glycol, dextran, and pullulan.

13. The method of any of the preceding claims, wherein the detector is a fluorescence detector, such as a laser induced fluorescence (LIF) detector, lamp-based fluorescence detector, or native fluorescence detector.

14. The method of any of the preceding claims, wherein the limit of quantification is in a low ng/mL range or a sub ng/mL range.

15. The method of any of the preceding claims, wherein at least two labeled viral protein components are prepared, alternatively at least three labeled viral protein components are prepared, alternatively at least four labeled viral protein components are prepared, alternatively at least five labeled viral protein components are prepared, alternatively at least six labeled viral protein components are prepared, alternatively at least seven labeled viral protein components are prepared, alternatively at least eight labeled viral protein components are prepared, alternatively at least nine labeled viral protein components are prepared, alternatively at least ten labeled viral protein components are prepared.

## Patentansprüche

1. Verfahren zum Quantifizieren einer Virusspezies, wobei das Verfahren die folgenden Schritte umfasst:
Herstellen mindestens einer markierten Virusproteinkomponente durch Inkubieren eines nachweisbaren Farbstoffs mit einer Virusspezies in Gegenwart von Natriumdodecylsulfat (SDS);
Laden der markierten Virusproteinkomponente auf eine Kapillarelektrophorese (CE)-Kapillare, wobei die CE-Kapillare mit einem Puffer gefüllt ist, der eine Polymermatrix umfasst:
Anlegen einer Trennspannung an die CE-Kapillare;
Nachweisen mindestens einer markierten Virusproteinkomponente mit einem Detektor, wodurch ein entsprechender Satz von Werten erzeugt wird; und
Quantifizieren eines Proteins von Interesse in der Virusproteinkomponente unter Verwendung des entsprechenden Wertesatzes,
**dadurch gekennzeichnet, dass** die Virusspezies ein Retrovirus ist.

2. Verfahren nach Anspruch 1, wobei die Virusspezies ein Lentivirus ist.

3. Verfahren nach Anspruch 2, wobei die Virusproteinkomponente ein Strukturprotein, ein Nichtstrukturprotein oder ein Restprotein ist.

4. Verfahren nach Anspruch 3, wobei die Virusproteinkomponente ein Strukturprotein ist, das aus der Gruppe bestehend aus Nukleokapsidprotein, Kapsidprotein, Matrixprotein und Hüllprotein ausgewählt ist.

5. Verfahren nach Anspruch 3 oder Anspruch 4, wobei das Strukturprotein ein p24-Kapsidprotein ist.

6. Verfahren nach einem der vorstehenden Ansprüche, wobei die Virusspezies bei einer Temperatur zwischen etwa 40 °C bis etwa 90 °C, alternativ bei einer Temperatur zwischen etwa 45 °C bis etwa 85 °C, alternativ bei einer Temperatur zwischen etwa 50 °C bis etwa 80 °C, alternativ bei einer Temperatur zwischen etwa 55 °C bis etwa 78 °C, alternativ bei einer Temperatur zwischen etwa 60 °C bis etwa 77 °C, alternativ bei einer Temperatur zwischen etwa 65 °C bis etwa 75 °C, alternativ bei einer Temperatur zwischen etwa 68 °C bis etwa 74 °C, alternativ bei einer Temperatur zwischen etwa 69 °C und etwa 73 °C, alternativ bei einer Temperatur von etwa 70 °C, inkubiert wird.

7. Verfahren nach einem der vorstehenden Ansprüche, wobei die Virusspezies für mindestens 2 Minuten, alternativ mindestens 5 Minuten, alternativ mindestens 10 Minuten, alternativ mindestens 15 Minuten, alternativ mindestens 20 Minuten, alternativ mindestens 25 Minuten, alternativ mindestens 30 Minuten, alternativ mindestens 35 Minuten, alternativ mindestens 40 Minuten, alternativ mindestens 45 Minuten, alternativ mindestens 50 Minuten, alternativ mindestens 55 Minuten, alternativ mindestens 60 Minuten, inkubiert wird.

8. Verfahren nach einem der vorstehenden Ansprüche, wobei der nachweisbare Farbstoff ein Fluoreszenzfarbstoff ist.

9. Verfahren nach Anspruch 8, wobei der nachweisbare Farbstoff eine Absorptionswellenlänge und eine Emissionswellenlänge zwischen etwa 480 nm und etwa 750 nm aufweist.

10. Verfahren nach Anspruch 8 oder Anspruch 9, wobei der nachweisbare Farbstoff ein Farbstoff auf Pyryliumbasis ist.

11. Verfahren nach Anspruch 10, wobei der nachweisbare Farbstoff ein aminreaktiver Pyrylium-Farbstoff ist.

12. Verfahren nach einem der vorstehenden Ansprüche, wobei die Polymermatrix aus der Gruppe bestehend aus vernetztem Polymer, linearen Polymeren, leicht verzweigten Polymeren, linearem Polyacrylamid, Polyethylenoxid, Polyethylenglykol, Dextran und Pullulan ausgewählt ist.

13. Verfahren nach einem der vorstehenden Ansprüche, wobei der Detektor ein Fluoreszenzdetektor ist, wie z. B. ein Detektor für laserinduzierte Fluoreszenz (LIF), ein Detektor für Fluoreszenz auf Lampenbasis oder ein Detektor für native Fluoreszenz.

14. Verfahren nach einem der vorstehenden Ansprüche, wobei die Quantifizierungsgrenze in einem niedrigen ng/mL-Bereich oder einem sub-ng/mL-Bereich liegt.

15. Verfahren nach einem der vorstehenden Ansprüche, wobei mindestens zwei markierte virale Proteinkomponenten hergestellt werden, alternativ mindestens drei markierte virale Proteinkomponenten hergestellt werden, alternativ mindestens vier markierte virale Proteinkomponenten hergestellt werden, alternativ mindestens fünf markierte virale Proteinkomponenten hergestellt werden, alternativ mindestens sechs markierte virale Proteinkomponenten hergestellt werden, alternativ mindestens sieben markierte virale Proteinkomponenten hergestellt werden, alternativ mindestens acht markierte virale Proteinkomponenten hergestellt werden, alternativ mindestens neun markierte virale Proteinkomponenten hergestellt werden, alternativ mindestens zehn markierte virale Proteinkomponenten hergestellt werden.

## Revendications

1. Procédé destiné à quantifier une espèce virale, le procédé comprenant les étapes suivantes :
préparer au moins un composant de protéine virale marqué par l'incubation d'un colorant détectable
avec une espèce virale en présence de dodécylsulfate de sodium (SDS : sodium dodecyl sulfate) ;
charger le composant de protéine virale marqué sur un capillaire d'électrophorèse capillaire (CE), dans lequel le capillaire CE est rempli avec un tampon comprenant une matrice polymère ;
appliquer une tension de séparation au capillaire CE ;
détecter au moins un composant de protéine virale marqué avec un détecteur, ce qui produit un ensemble de valeurs correspondant ; et
quantifier une protéine d'intérêt dans le composant de protéine virale en utilisant l'ensemble de valeurs correspondant,
**caractérisé en ce que** l'espèce virale est un rétrovirus.

2. Procédé selon la revendication 1, dans lequel l'espèce virale est un lentivirus.

3. Procédé selon la revendication 2, dans lequel le composant de protéine virale est une protéine structurale, une protéine non structurale ou une protéine résiduelle.

4. Procédé selon la revendication 3, dans lequel le composant de protéine virale est une protéine structurale choisie dans le groupe consistant en la protéine de nucléocapside, la protéine de capside, la protéine de matrice et la protéine d'enveloppe.

5. Procédé selon la revendication 3 ou la revendication 4, dans lequel la protéine structurale est une protéine de capside p24.

6. Procédé selon l'une quelconque des revendications précédentes, dans lequel l'espèce virale est incubée à une température comprise d'environ 40°C à environ 90°C, en variante à une température comprise d'environ 45°C à environ 85°C, en variante à une température comprise d'environ 50°C à environ 80°C, en variante à une température comprise d'environ 55°C à environ 78°C, en variante à une température comprise d'environ 60°C à environ 77°C, en variante à une température comprise d'environ 65°C à environ 75°C, en variante à une température comprise d'environ 68°C à environ 74°C, en variante à une température comprise d'environ 69°C à environ 73°C, en variante à une température d'environ 70°C.

7. Procédé selon l'une quelconque des revendications précédentes, dans lequel l'espèce virale est incubée pendant au moins 2 minutes, en variante au moins 5 minutes, en variante au moins 10 minutes, en variante au moins 15 minutes, en variante au moins 20 minutes, en variante au moins 25 minutes, en variante au moins 30 minutes, en variante au moins 35 minutes, en variante au moins 40 minutes, en variante au moins 45 minutes, en variante au moins 50 minutes, en variante au moins 55 minutes, en variante au moins 60 minutes.

8. Procédé selon l'une quelconque des revendications précédentes, dans lequel le colorant détectable est un colorant fluorescent.

9. Procédé selon la revendication 8, dans lequel le colorant détectable présente une longueur d'onde d'absorption et une longueur d'onde d'émission comprises entre environ 480 nm et environ 750 nm.

10. Procédé selon la revendication 8 ou la revendication 9, dans lequel le colorant détectable est un colorant à base de pyrylium.

11. Procédé selon la revendication 10, dans lequel le colorant détectable est un colorant de pyrylium réactif aux amines.

12. Procédé selon l'une quelconque des revendications précédentes, dans lequel la matrice polymère est choisie dans le groupe consistant en polymère réticulé, polymères linéaires, polymères légèrement ramifiés, polyacrylamide linéaire, oxyde de polyéthylène, polyéthylène glycol, dextrane et pullulane.

13. Procédé selon l'une quelconque des revendications précédentes, dans lequel le détecteur est un détecteur de fluorescence, tel qu'un détecteur de fluorescence induite par laser (LIF), un détecteur de fluorescence basé sur une lampe ou un détecteur de fluorescence native.

14. Procédé selon l'une quelconque des revendications précédentes, dans lequel la limite de quantification est dans une plage basse de ng/mL ou une sub-plage de ng/mL.

15. Procédé selon l'une quelconque des revendications précédentes, dans lequel au moins deux composants de protéine virale marqués sont préparés, en variante au moins trois composants de protéine virale marqués sont préparés, en variante au moins quatre composants de protéine virale marqués sont préparés, en variante au moins cinq composants de protéine virale marqués sont préparés, en variante au moins six composants de protéine virale marqués sont préparés, en variante au moins sept composants de protéine virale marqués sont préparés, en variante au moins huit composants de protéine virale marqués sont préparés, en variante au moins neuf composants de protéine virale marqués sont préparés, en variante au moins dix composants de protéine virale marqués sont préparés.
